# EUROPÄISCHE PATENTSCHRIFT

(11) **EP 0 532 835 B1**
(45) Veröffentlichungstag und Bekanntmachung des Hinweises auf die Patenterteilung: **15.04.1998**
(21) Anmeldenummer: 92109430.6
(22) Anmeldetag: 04.06.1992
(51) Int. Cl.: A61M 1/16, A61J 1/00

(54) **Kartusche eines medizinischen Gerätes, insbesondere eines Dialysegerätes**
Cartridge for medical apparatus, especially for dialysis apparatus
Cartouche pour appareil médical, notamment pour appareil de dialyse

(30) Priorität: 15.09.1991 DE 4130412
(43) Veröffentlichungstag der Anmeldung: 24.03.1993
(73) Patentinhaber: Zaunbauer, Peter, Dr., D-30519 Hannover (DE)
(72) Erfinder: Zaunbauer, Peter, Dr.med., W-3000 Hannover 81 (DE); Volkmann, Helmut, Dr.Ing., Dr.med., W-3057 Neustadt am Rübenberge (DE)
(74) Vertreter: Junius, Walther, Dr.

(56) Entgegenhaltungen:
- EP-A- 0 278 100
- BE-A- 664 031
- DE-A- 3 508 915
- DE-U- 9 111 524
- US-A- 4 966 512

## Beschreibung

Die Erfindung betrifft eine Kartusche für die Lösung einer pulverförmigen oder körnigen festen Substanz in einem die Kartusche durchlaufenden Lösungsmittel, wie sie im Oberbegriff des Hauptanspruches beschrieben ist.

Kartuschen dieser Art, die zur Lösung eines Wirkstoffes in Wasser verwendet werden, sind für unterschiedliche Einsatzzwecke bekannt geworden: Im Falle der DE-A- 35 08 915 zur Lösung von Düngemitteln in Wasser, um mit einem Gartenschlauch eine sehr verdünnte Düngemittellösung auszutragen, im Falle der BE-A-664 031 zur Zufügung von Substanzen zu Wasser, und im Falle der EP 0 278 l00 A2 für die Verwendung in medizinischen Geräten.

Um die Verwendung in medizinischen Geräten geht es auch bei der vorliegenden Erfindung.

Die Kartuschen der EP 0 278 l00 A2 für die Verwendung in medizinischen Geräten werden aus Kunststoff als einstückiges Bauteil gefertigt. In einer Ausführungsform ist ein zylindrisches Rumpf des Gefäßes mit Deckel und Boden fest verbunden, wobei der zylindrische Rand von Deckel und Boden den zylindrischen Rumpf umfaßt. Filter sind zwischen den Stirnseiten des Rumpfes und Deckel bzw.Boden gehaltert. In einer anderen Ausführungsform ist der Rumpf kegelstumpfförmig einstückig mit einem Bodenteil gestaltet. Der gesondert von dem Bauteil Behälter-Boden hergestellte Deckel wird nach der Befüllung des Behälters mit der pulverförmigen oder körnigen Substanz mit dem oberen Rand des Behälters fest durch Verschweißen oder Verkleben verbunden. Diese bekannte Kartusche wird nach einmaligem Gebrauch weggeworfen.

Dadurch wird nicht nur Kunststoff vergeudet, sondern es wird auch die Umwelt belastet. Denn es werden die im medizinischen Betrieb anfallenden, nur mit erheblichem Aufwand und Schwierigkeiten zu entsorgenden Abfallmengen vergrößert. Diese weggeworfenen Kartuschen bereiten nicht nur vom Volumen her Probleme, sondern auch von der Art der Abfallbeseitigung. Die in der Kartusche befindliche verwendete Substanz wird in vielen Fällen nicht vollständig verbraucht, sondern es bleibt oftmals ein Rest in der Kartusche, der selbst dann, wenn die Substanz in der Müllverbrennung unproblematisch ist, die Müllverbrennung durch die in der Substanz verbliebene Feuchtigkeit behindert.

Aber auch wirtschaftlich ist die Verwendung von Wegwerfkartuschen nicht günstig, da die Herstellung dieser Kartuschen und ihr festes Verschließen einen erheblichen Kostenfaktor bei der Herstellung bilden.

Darüber hinaus kann unter Umständen die Verwendung von Wegwerfkartuschen auch aus medizinischer Sicht unvorteilhaft sein, nämlich dann, wenn durch das neue Material, z.B. durch in winzigen Mengen an der Innenoberfläche der Kartusche verbliebene Hilfsmittel zur Entformung im Herstellungsprozeß, Immunreaktionen des Patienten ausgelöst werden, die nicht ausgelöst würden, wenn das Kunststoffmaterial mehrmals benutzt worden wäre. Solche Reaktionen sind beispielsweise von Dialyseatoren her bekannt.

Die nicht für medizinische Zwecke bestimmten und auch nicht geeigneten Kartuschen der BE-A-664 031 und der DE -A-35 08 915 vermögen keine Anregungen zu geben, um die genannten Probleme zu lösen. Sie sind zwar wiederverwendbar, jedoch aus vielen schwer zu reinigenden Teilen zusammengebaut. Im Falle der DE-A-35 08 915 sind in die Kartusche aus Kunststoff hergestellte Wirkstoffpatronen einzulegen, deren Entsorgung die gleichen Probleme wie die Entsorgung der ganzen Kartuschen der EP-A-0 278 100 bereitet.

Die Erfindung vermeidet die Nachteile des Standes der Technik. Es ist die Aufgabe der vorliegenden Erfindung, eine einfach aufgebaute, preiswert herstellbare, individuell füllbare, leicht zu desinfizierende Kartusche für einen vielmaligen Gebrauch zu schaffen.

Die erfindungsgemäße Kartusche ist im kennzeichnenden Teil des Hauptanspruches beschrieben.

Die erfindungsgemäße Kartusche ist aus nur wenigen Teilen aufgebaut, einem kegelstumpfförmigen Behälter, einem Deckel, einem Boden, einem Filter und eventuell zwei Dichtringen. Diese Teile sind leicht auseinanderzubauen, zu reinigen bzw. zu desinfizieren und wieder zusammenzubauen. Es ist ein Vorteil dieser Kartusche, daß weitere Klemm- und Befestigungsmittel nicht benötigt werden. Durch die in so einfacher Weise ausgeführte lösbare Befestigung sowohl des Bodens als auch des Deckels wird auch erreicht, daß die Kartusche leicht wiederzubefüllen ist. Die feste Substanz wird dabei zweckmäßigerweise in Folienbeuteln angeliefert. Der wegzuwerfende Beutel besteht nur aus einem Bruchteil desjenigen Kunststoffes, der sonst für die Wegwerfkartusche benutzt wird. Die Beseitigung dieses Beutels in der Müllverbrennung ist problemlos, weil die dem Beutel entnommene Substanz in trockenem Zustand und vollständig entnommen worden ist.- Durch den abschraubbaren Boden ist das Filter leicht zugänglich. Dieses Filter sitzt in der hinterschnittenen Ausnehmung sehr fest. Es kann daher z.B. durch Rückspülung leicht gereinigt werden. Ist es einmal nach vielmaligem Gebrauch der Kartusche auszuwechseln, so kann dieses sehr leicht durch Ausstoßen des Filters aus der hinterschnittenen Ausnehmung erfolgen. Durch den Sitz in der hinterschnittenen Ausnehmung ist gesichert, daß seitlich am Filter keine Festsubstanz vorbeigelangen kann.

Zweckmäßig ist es, wenn Dichtringe für die Abdichtung des in die Kartusche eingesetzten Bodens und Deckels vorhanden sind. Durch derartige Abdichtungen mit Dichtringen wird eine sehr zuverlässige Abdichtung erzielt.

Für eine gute Abdichtung kann es vorteilhaft sein, wenn an der Kartusche eine senkrecht zur Achse verlaufende Dichtfläche von der Unterfläche aus gesehen hinter dem Gewinde und am Boden eine Ausnehmung für die Aufnahme des Dichtringes an der in die Kartusche eintauchenden Stirnseite vorgesehen ist.

Aber nicht nur mit Dichtringen ist eine zuverlässige Abdichtung möglich, sondern auch dadurch, daß der Boden aus Kunststoff hergestellt ist, daß der Boden einen im Querschnitt halbkreisförmigen Vorsprung versehen ist, der eine gewisse Eigenelastizität aufweist, und daß die Dichtfläche des Behälters an diesem Vorsprung anliegt.

Insbesondere kann es vorteilhaft sein, wenn an dem Behälter oberhalb des an seinem unteren Ende angeordneten Gewindes eine senkrecht zur Behälterachse verlaufende Dichtfläche und am Boden an der in den Behälter eintauchenden Stirnseite ein im Querschnitt halbkreisförmiger, trapezförmiger oder dreieckförmiger Vorsprung vorgesehen sind.

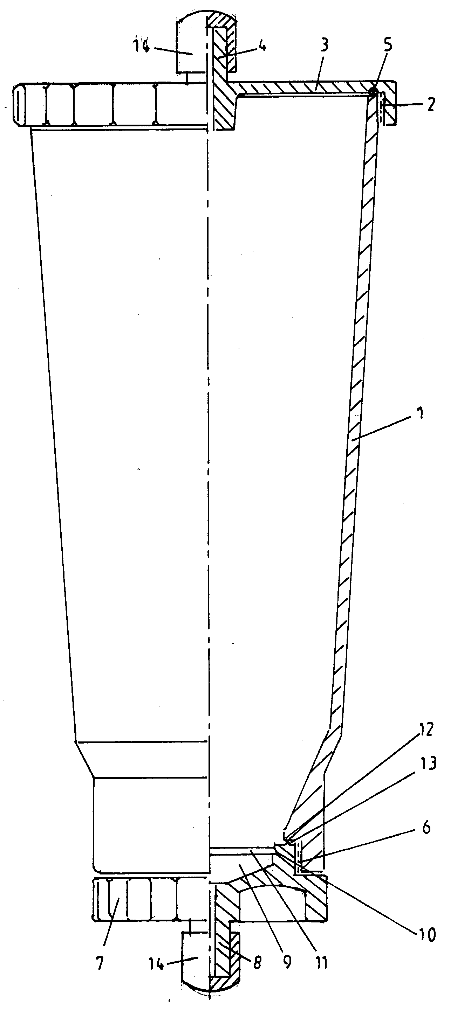

Das Wesen der Erfindung ist nachstehend anhand eines in der Zeichnung schematisch dargestellten Ausführungsbeispieles näher erläutert. Die Zeichnung zeigt die Kartusche links in Ansicht, rechts im Querschnitt.

Der leicht konisch gestaltete Behälter 1 weist an seinem oberen Ende ein Außengewinde 2 auf, auf welches der mit Innengewinde versehene Deckel 3 aufgeschraubt ist. Dieser Deckel 3 weist zentrisch einen Anschlußstutzen 4 auf. Ein Dichtring 5 ist in eine Nut im Deckel 3 eingelegt. Im aufgeschraubten Zustand liegt dieser Dichtring 5 auf der Stirnseite des Behälters 1.

Am unteren Ende weist der Behälter 1 ein Innengewinde 6 auf, in welches der Boden 7 eingeschraubt ist. Der Boden 7 weist zentrisch einen Auslaufstutzen 8 auf und ist auf der dem Innenraum zugewandten Seite wannenförmig gestaltet. Oberhalb dieser Wanne 9 befindet sich eine ringförmige hinterschnittene Ausnehmung 10, in die das Filter 11 eingesprengt ist. Der Behälter 1 weist oberhalb des Gewindes 6 eine Dichtfläche 12 auf, gegen welche der ringartige, im Querschnitt halbkreisförmige Vorsprung 13 anliegt. Da dieser Vorsprung 13 wie der gesamte Boden aus Kunststoff hergestellt ist und eine gewisse Eigenelastizität hat, wirkt er wie ein Dichtring.

Der Einlaufstutzen 4 und der Auslaufstutzen 8 sind mit Kappen 14 während des Nichtgebrauches verschlossen, um das Eindringen von Staub oder dergleichen während der Lagerung der Kartusche im leeren oder gefüllten Zustand zu verhindern.

Es ist zweckmäßig, für ein medizinisches Gerät mehrere dieser wiederverwendbaren Kartuschen auf Lager liegen zu haben, die nach der Reinigung gleich wieder mit der benötigten Substanz gefüllt werden. So stehen jederzeit griffbereit mehrere auswechselbare Kartuschen zur Verfügung.

Anstelle des Vorsprunges 13 kann in einer ringförmigen Rille an der in den Behälter 1 eintauchenden Stirnseite auch ein Dichtring 5 angeordnet sein. Verständlicherweise kann die Abdichtung aber auch in kinematischer Umkehr dadurch erfolgen, daß die Dichtringe in Rillen des Behälters untergebracht sind und korrespondierende Dichtflächen am Deckel und am Boden vorgesehen sind. Es besteht aber ebenso die weitere Möglichkeit, die Abdichtung mit Hilfe von Konusflächen zu erreichen, die einerseits am Behälter, andererseits am Deckel und/oder Boden angeordnet sind.

Einer der entscheidenden Vorteile der Erfindung gegenüber der Wegwerfkartusche besteht darin, daß die wiederbenutzbare Kartusche den behandelnden Arzt in die Lage versetzt, die Dauer der jeweiligen Dialyse durch individuelle Füllung der Kartusche jeweils den individuellen medizinischen Bedürfnissen des einzelnen Patienten zu bestimmen. In der medizinischen Praxis hat sich gezeigt, daß die Dialysedauer je nach der Restfunktion der Nieren eines jeden Patienten und je nach den sonstigen medizinischen Vorgaben zwischen zwei und sechs Stunden liegt. Während die Wegwerfkartusche generell von einer Dialysedauer von fünf Stunden ausgeht und damit an den Bedürfnissen der Praxis vorbeigeht, ermöglicht die erfindungsgemäße Kartusche durch individuelle Quantifizierung der Pulvermenge die zu verwendende Kartusche der Dialysedauer anzupassen.

## Patentansprüche

1. Kartusche eines medizinischen Gerätes, insbesondere eines Dialysegerätes, für die Lösung einer pulverförmigen oder körnigen festen Substanz in einem die Kartusche durchlaufenden Lösungsmittel, bestehend aus einem im wesentlichen kegelstumpfförmigen Behälter (1) für die Aufnahme der festen Substanz, mit einem im Bodenbereich angeordneten Filter (11), mit einem mit einem zentrisch angeordneten Zuflußstutzen (4) und mit einem zentrisch angeordneten Ausflußstutzen (8),
dadurch gekennzeichnet,
daß der Zuflußstutzen (4) an einem Deckel (3) vorgesehen ist, der abgedichtet und durch eine Schraubverbindung lösbar am Behälter (l) befestigt ist,
daß der Ausflußstutzen (8) in einem Boden (7) vorgesehen ist, der abgedichtet und durch eine Schraubverbindung lösbar am Behälter (l) befestigt ist,
daß sowohl der Boden (7) als auch der Deckel (3) mit Schraubgewinden (2,6) versehen sind und mit diesen an dem Behälter (1) lösbar befestigt sind
und daß das Filter (ll) mit seinem Rand in eine hinterschnittene Ausnehmung (l0) eingesprengt ist.

2. Kartusche nach Anspruch l,
dadurch gekennzeichnet,
daß der Behälter (1) an seinem oberen Ende ein Außengewinde (2) aufweist, auf welches der mit Innengewinde versehene Deckel (3) aufgeschraubt ist.

3. Kartusche nach Anspruch l,
dadurch gekennzeichnet,
daß der Behälter (1) am unteren Ende ein Innengewinde (6) aufweist, in welches der Boden (7) eingeschraubt ist, dessen zentrischer Auslaufstutzen (8) auf der dem Innenraum zugewandten Seite wannenförmig gestaltet ist.

4. Kartusche nach Anspruch l,
dadurch gekennzeichnet,daß ein Dichtring (5) für die Abdichtung des in den Behälter (l) eingesetzten Bodens (7) und/oder Deckels (3) vorgesehen ist.

5. Kartusche nach Anspruch l oder 2,
dadurch gekennzeichnet,
daß an dem Behälter (l) oberhalb des an seinem unteren Ende angeordneten Gewindes (6) eine senkrecht zur Behälterachse verlaufende Dichtfläche (l2) und am Boden (7) an der in den Behälter (l) eintauchenden Stirnseite eine Ausnehmung für die Aufnahme des Dichtringes vorgesehen sind.

6. Kartusche nach Anspruch l,
dadurch gekennzeichnet,
daß der Boden (7) aus Kunststoff hergestellt ist, daß der Boden (7) einen im Querschnitt halbkreisförmigen Vorsprung (13) versehen ist, der eine gewisse Eigenelastizität aufweist,
und daß die Dichtfläche (12) des Behälters (1) an diesem Vorsprung (13) anliegt.

7. Kartusche nach Anspruch 3,
dadurch gekennzeichnet,
daß an dem Behälter (l) oberhalb des an seinem unteren Ende angeordneten Gewindes (6) eine senkrecht zur Behälterachse verlaufende Dichtfläche (l2) und am Boden (7) an der in den Behälter (l) eintauchenden Stirnseite ein im Querschnitt halbkreisförmiger, trapezförmiger oder dreieckförmiger Vorsprung (l3) vorgesehen sind.

## Claims

1. Cartridge for a medical apparatus, in particular a dialysis apparatus, for dissolving a powdery or granular solid substance in a solvent running through the cartridge, consisting of a container (1) with the essential form of a truncated cone for accommodating the solid substance, with a filter (11) disposed in the bottom area, with a centrally disposed inlet connector (4) and with a centrally disposed outlet connector (8), characterised in that the inlet connector (4) is provided on a lid (3) which is sealed and secured detachably to the container (1) by a screwed connection, in that the outlet connector (8) is provided in a bottom (7) which is sealed and secured detachably to the container (1) by a screwed connection, in that both the bottom (7) and the lid (3) are provided with screw threads (2, 6) and are detachably secured to the container (1) with these and in that the filter (11) is snap-fitted by its edge into an undercut recess (10).

2. Cartridge according to claim 1, characterised in that at its upper end the container (1) exhibits an external thread (2) onto which the lid (3) provided with an internal thread is screwed.

3. Cartridge according to claim 1, characterised in that at the lower end the container (1) exhibits an internal thread (6) into which the bottom (7) is screwed, the central outlet connector (8) of which is cup-shaped on the side facing the interior.

4. Cartridge according to claim 1, characterised in that a sealing ring (5) is provided for sealing the bottom (7) and/or lid (3) fitted in the container (1).

5. Cartridge according to claim 1 or 2, characterised in that a sealing face (12) running perpendicular to the axis of the container is provided on the container (1) above the thread (6) disposed at its lower end, and a recess for accommodating the sealing ring is provided on the bottom (7) on the end side engaging in the container (1).

6. Cartridge according to claim 1, characterised in that the bottom (7) is manufactured of plastic, in that the bottom (7) is provided with a projection (13) of semicircular cross-section which exhibits a certain inherent elasticity, and in that the sealing face (12) of the container (1) bears on this projection (13).

7. Cartridge according to claim 3, characterised in that a sealing face (12) running perpendicular to the axis of the container is provided on the container (1) above the thread (6) disposed at its lower end, and a projection (13) of semicircular, trapezoidal or triangular cross-section is provided on the bottom (7) on the end side engaging in the container (1).

## Revendications

1. Cartouche d'un appareil médical, en particulier d'un appareil de dialyse, pour la mise en solution d'une substance solide, pulvérulente ou granuleuse, dans un solvant qui traverse la cartouche, constituée d'un récipient (1), sensiblement en forme de tronc de cône, pour recevoir la substance solide, comportant un filtre (11) disposé dans la zone du fond, comportant une tubulure d'entrée (4) disposée au centre et une tubulure de sortie (8) disposée au centre,
caractérisée par le fait
■ que la tubulure d'entrée (4) est prévue sur un couvercle (3) qui est rendu étanche et qui est fixé, de façon amovible, au récipient (1) par une liaison par vissage,
■ que la tubulure de sortie (8) est prévue dans un fond (7) qui est rendu étanche et qui est fixé, de façon amovible, au récipient (1) par une liaison par vissage,
■ qu'aussi bien le fond (7) que le couvercle (3) sont munis de filetages (2, 6) et sont, par eux, fixés de façon amovible, au récipient (1) et que le filtre (11) est forcé, par son bord, dans un évidement en contre-dépouille (10).

2. Cartouche selon la revendication 1,
caractérisée par le fait
■ que le récipient (1) présente à son extrémité supérieure, un filetage extérieur (2) sur lequel se visse le couvercle (3) muni d'un filetage intérieur.

3. Cartouche selon la revendication 1,
caractérisée par le fait
■ que le récipient (1) présente, à son extrémité inférieure, un filetage intérieur (6) sur lequel se visse le fond (7) dont la tubulure centrale de sortie (8) a la forme d'une cuvette du côté tourné vers l'espace intérieur.

4. Cartouche selon la revendication 1,
caractérisée par le fait
■ qu'est prévue une bague d'étanchéité (5) pour l'étanchéité du fond (7) et/ou du couvercle (3) placé sur le récipient (1).

5. Cartouche selon la revendication 1 ou 2,
caractérisée par le fait
■ que sur le récipient (1) sont prévus, au-dessus du filetage (6) disposé à son extrémité inférieure, une surface d'étanchéité (19) orientée perpendiculairement à l'axe du récipient et, au fond (7), sur la face frontale qui pénètre dans le récipient (1), un évidement pour recevoir la bague d'étanchéité.

6. Cartouche selon la revendication 1,
caractérisée par le fait
■ que le fond (7) est fabriqué en plastique, que le fond (7) présente un saillant (13), en forme de demi-cercle en coupe, qui présente une certaine élasticité propre, et que la surface d'étanchéité (12) du récipient (1) s'appuie contre ce saillant (13).

7. Cartouche selon la revendication 3,
caractérisée par le fait
■ que sur le récipient (1) sont prévus, au-dessus du filetage (6) disposé à son extrémité inférieure, une surface d'étanchéité (12) orientée perpendiculairement à l'axe du récipient et, au fond (7), sur la face frontale qui pénètre dans le récipient (1), un saillant (13) ayant en coupe la forme d'un demi-cercle, d'un trapèze ou d'un triangle.
